(19) Europäisches Patentamt
European Patent Office
Office européen des brevets

(11) Publication number: 0 143 281 B1

(12) # EUROPEAN PATENT SPECIFICATION

(45) Date of publication of patent specification: 31.10.90

(51) Int. Cl.$^5$: A 01 N 43/58

(21) Application number: 84111895.3

(22) Date of filing: 29.09.81

(60) Publication number of the earlier application in accordance with Art. 76 EPC: 0 049 971

(54) **Substituted pyridazines, processes for making them, their use as plant growth regulators, and plant growth regulating compositions containing them.**

(30) Priority: 03.10.80 US 193672

(43) Date of publication of application: 05.06.85 Bulletin 85/23

(45) Publication of the grant of the patent: 31.10.90 Bulletin 90/44

(84) Designated Contracting States: AT BE CH DE FR GB IT LI NL SE

(56) References cited:
FR-A-2 383 605
FR-A-2 392 980

Monatshefte der Chemie 91, 1165 (1960)

(73) Proprietor: IMPERIAL CHEMICAL INDUSTRIES PLC
Imperial Chemical House, Millbank
London SW1P 3JF (GB)

(72) Inventor: Patterson, Dennis Ray
407 Washington Avenue
North Wales Pennsylvania 19454 (US)

(74) Representative: Arnold, Graham Donald et al
Imperial Chemical Industries PLC Legal
Department: Patents PO Box 6
Welwyn Garden City Herts, AL7 1HD (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).



Courier Press, Leamington Spa, England.

**Description**

This invention concerns novel substituted pyridazines and their use as plant growth regulators, particularly as chemical sterilants for producing hybrid seed of cereal grain plants; also concerned are processes for making the new compounds and plant growth regulating compositions containing them.

In Belgian Patent specification No. 864,704 and its foreign equivalents: French No. 2,392,980, W. German No. 2,808,795, Egyptian No. 13401, Australian No. 78/33980 and New Zealand No. 186,596 there are described 1-aryl(or substituted aryl)-1,4-dihydro-4-oxo-3-carboxy-6-alkyl-pyridazines and derivatives thereof. These compounds are disclosed as having utility as plant growth regulators particularly as chemical hybridizing agents for the production of hybrid cereal seed.

The present invention provides novel substituted pyridazines which bear some resemblance to those of the prior art compounds noted above but which are principally characterized in that they contain a carboxy group, or derivative thereof, in the 5-position and in the 3-position as well.

The pyridazine compounds concerned in this invention are those of Formula (I) below.

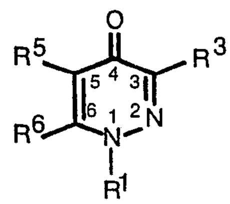

(I)

wherein $R^1$ is $(C_1—C_4)$alkyl, phenyl or naphthyl each of the last two groups being optionally substituted with up to three (preferably one or two) of the same or different substituents selected from halogen (viz. chlorine, bromine, fluorine and iodine), trihalomethyl (e.g. trifluoromethyl or trichloromethyl), $(C_1—C_4)$alkoxy, $(C_1—C_4)$alkyl and cyano;

$R^3$ is a group as defined below for $R^5$;

$R^5$ is a carboxy (—COOH) group or agronomically acceptable alkali metal salt thereof (particularly a salt of Na, K or Li), an alkoxycarbonyl (—COOR) group or a substituted carbamoyl (—CONR′R″) group, wherein R represents $(C_1—C_4)$alkyl, R′ represents hydrogen or $(C_1—C_4)$alkyl and R″ represents $(C_1—C_4)$ alkyl; and

$R^6$ is a $(C_1—C_4)$ alkyl group or an optionally substituted phenyl or naphthyl group as defined for $R^1$ above: provided where $R_1$ is unsubstituted phenyl $R_6$ is not 2-methoxyphenyl.

The compound 1-phenyl-1,4-dihydro-4-oxo-6-(2-methoxyphenyl)-3,5 dicarboxylic acid (monomethyl ester) is disclosed in Monatshefte der Chemie *91,* 1165 (1960). Accordingly we do not claim this compound *per se.*

Compounds of Formula (I) meriting particular attention are those wherein $R^1$ is halophenyl, particularly 4-halo-phenyl, $R^3$ is $—CO_2Na$, $—CO_2K$ or $—CO_2(C_1—C_3)$ alkyl, $R^5$ is $—CO_2CH_3$ and $R^6$ is methyl or ethyl.

The term alkyl used in this specification includes both straight and branched chain alkyl groups and, where unqualified, alkyl means n-alkyl.

Also included within the scope of the invention are agronomically acceptable acid addition salts of compounds of Formula (I). Typical acid addition salts are those formed with strong acids such as hydrochloric, hydrobromic sulfuric and nitric acids.

Typical compounds of Formula (I) are:

1-phenyl-1,4-dihydro-4-oxo-6-methylpyridazine-3,5-dicarboxylic acid,
1-phenyl-1,4-dihydro-4-oxo-6-ethylpyridazine-3,5-dicarboxylic acid,
1-phenyl-1,4-dihydro-4-oxo-6-propylpyridazine-3,5-dicarboxylic acid,
1-phenyl-1,4-dihydro-4-oxo-6-butylpyridazine-3,5-dicarboxylic acid,
1-phenyl-1,4-dihydro-4-oxo-6-phenylpyridazine-3,5-dicarboxylic acid,
1-(4-chlorophenyl)-1,4-dihydro-4-oxo-6-methylpyridazine-3,5-dicarboxylic acid,
1-(4-bromophenyl)-1,4-dihydro-4-oxo-6-ethylpyridazine-3,5-dicarboxylic acid,
1-(3,4-dichlorophenyl)-1,4-dihydro-4-oxo-6-propylpyridazine-3,5-dicarboxylic acid,
1-(4-iodophenyl)-1,4-dihydro-4-oxo-6-butylpyridazine-3,5-dicarboxylic acid,
1-(4-fluorophenyl)-1,4-dihydro-4-oxo-6-phenylpyridazine-3,5-dicarboxylic acid,
1-(4-chlorophenyl)-1,4-dihydro-4-oxo-6-phenylpyridazine-3,5-dicarboxylic acid,
1-(3-chlorophenyl)-1,4-dihydro-4-oxo-6-methylpyridazine-3,5-dicarboxylic acid,
1-(2-chlorophenyl)-1,4-dihydro-4-oxo-6-ethylpyridazine-3,5-dicarboxylic acid,
1-(3-bromophenyl)-1,4-dihydro-4-oxo-6-propylpyridazine-3,5-dicarboxylic acid,
1-(2-bromophenyl)-1,4-dihydro-4-oxo-6-butylpyridazine-3,5-dicarboxylic acid,
1-(2,4,6-trichlorophenyl)-1,4-dihydro-4-oxo-6-benzylpyridazine-3,5-dicarboxylic acid,
1-(4-methylphenyl)-1,4-dihydro-4-oxo-6-phenylpyridazine-3,5-dicarboxylic acid,
1-(4-trifluoromethylphenyl)-1,4-dihydro-4-oxo-6-methylpyridazine-3,5-dicarboxylic acid,
1-(3-ethoxyphenyl)-1,4-dihydro-4-oxo-6-ethylpyridazine-3,5-dicarboxylic acid,
1-methyl-1,4-dihydro-4-oxo-6-propyl-pyridazine-3,5-dicarboxylic acid,

1-(3-cyanophenyl)-1,4-dihydro-4-oxo-6-butylpyridazine-3,5-dicarboxylic acid,
1-butyl-1,4-dihydro-4-oxo-6-methyl-pyridazine-3,5-dicarboxylic acid,
1-(2-trifluoromethyl-4-chlorophenyl)-1,4-dihydro-4-oxo-6-methylpyridazine-3,5-dicarboxylic acid,
1-(2-trifluoromethyl-4-bromophenyl)-1,4-dihydro-4-oxo-6-ethylpyridazine-3,5-dicarboxylic acid,
1-(2-chloro-5-trifluoromethylphenyl)-1,4-dihydro-4-oxo-6-ethylpyridazine-3,5-dicarboxylic acid,
1-(2-naphthyl)-1,4-dihydro-4-oxo-6-butylpyridazine-3,5-dicarboxylic acid,

and derivatives of each of the above listed compounds wherein the hydrogen atom in one or both carboxy groups is replaced by a group or atom selected from sodium, potassium, lithium, methyl, ethyl, n-propyl isopropyl and butyl (all isomers). There should also be listed the acid addition salts of all the preceding compounds in the above list.

The following is a sequence utilized to prepare the compounds concerned in this invention.

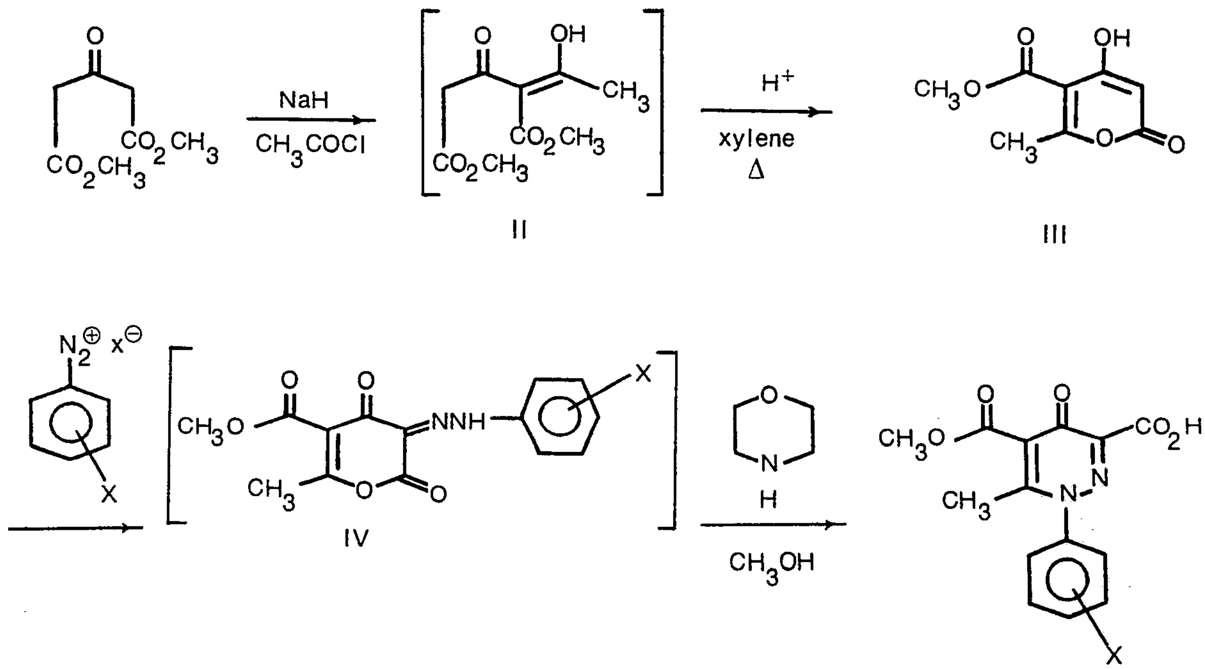


II III IV V

The synthetic sequence outlined above is unique in its ability to produce (V). No directed syntheses of either of these classes of compounds has been reported in the literature.

The reactions involving the sodium hydride and acetyl chloride can be carried out in an inert solvent at a temperature from about 0° to about 10°C; and the reaction of the pyrone (III) with the diazonium salt can be carried out at a temperature from about 0° to about 50°C.

In the above reaction sequence a 3-oxoglutarate is first reacted with sodium hydride thereby replacing a hydrogen atom from the active methylene group followed by reaction with acetyl chloride to form the intermediate of Formula II which then rearranges under acid conditions to form the pyrone of Formula III.

The pyrone is then reacted with a diazonium salt to form a hydrazone of Formula IV. The hydrazone is then rearranged in the presence of a base to form the dicarboxypyradazine of Formula V.

A more preferred synthetic route to compounds of Formula (I) is outlined below.

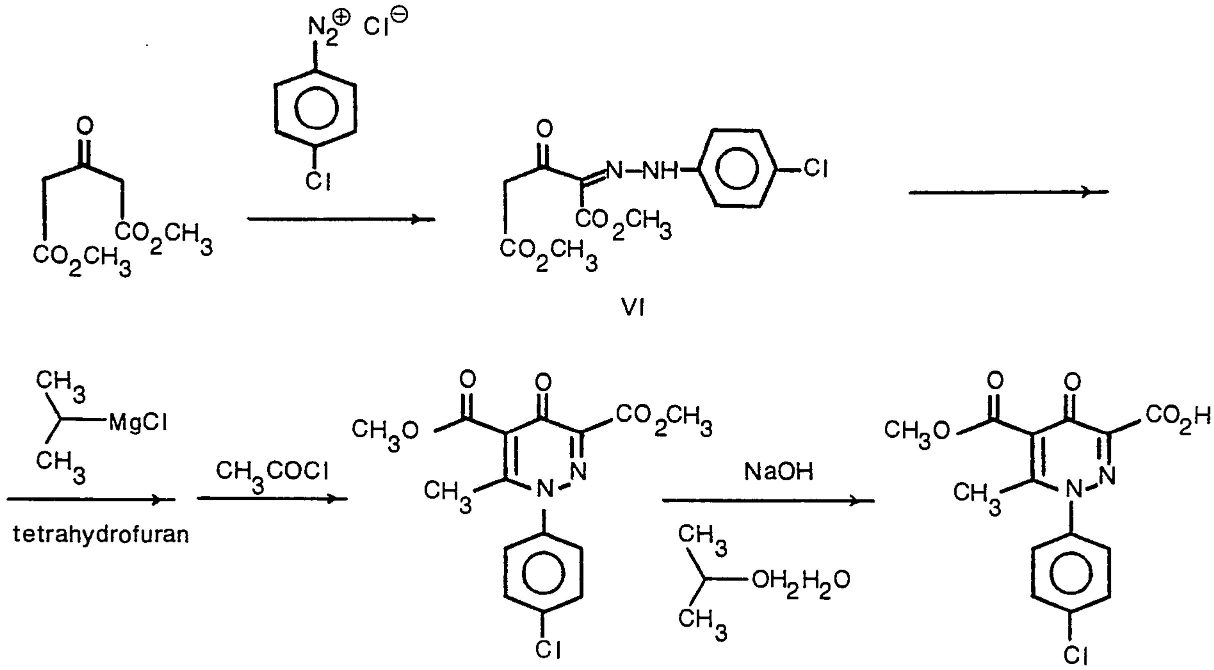


VI, VII, VIII

In this approach the 3-oxoglutarate is first reacted with a diazonium salt to form the hydrazone of Formula VI which is then reacted with isopropyl magnesium chloride followed by reaction with acetyl chloride to form the dicarboxypyridazine ester of Formula VII which is then hydrolyzed to the corresponding acid of Formula VIII.

The following examples are provided to illustrate the processes for preparing the compounds of Formula I. There may also be used other processes described in the literature for the preparation of analogous compounds such as processes described in Belgian Patent No. 864,704 and its foreign equivalents cited earlier. In the following preparations temperatures are in degrees Celsius and percentages are on a weight basis unless otherwise specified.

Example 1

Synthesis of 1-(p-Chlorophenyl)-1,4-dihydro-4-oxo-3-carboxy-5-methoxycarbony-6-methylpyridazine

A 250 ml, three necked, round bottomed flask was equipped with addition funnel, paddle stirrer and thermometer. The flask was charged with 50 ml methanol, sodium acetate (16.0 g, 0.198 mole), and the above pyrone of formula III (8.0 g, 0.043 mole). p-Chlorobenzenediazonium chloride was prepared on the side by the dropwise addition of sodium nitrite (3.3 g, 0.047 mole) in 10 ml water to a cooled (5°) slurry containing p-chloroaniline (5.6 g, 0.043 mole) in aqueous hydrochloric acid (16.5 ml 12N HCl [0.198 mole] plus 10 ml water). The diazonium chloride solution was added dropwise to the solution containing the pyrone. This addition was carried out during 10 min with no noticeable exotherm. After complete addition, the resulting orange slurry was stirred for 40 min at room temperature. Suction filtration gave an orange filter cake which was washed repeatedly with water, then sucked dry during 2 hr. The filter cake thus obtained was placed back into the three necked flask used above. Methanol (200 ml) was added to give a slurry. Morpholine (10.0 g, 0.115 mole) was added in one portion. A mildly exothermic reaction occurred, and a dark, homogeneous solution was obtained. After stirring 10 min the solution was poured into 300 ml water. This was extracted with methylene chloride (3 KS 100 ml). The combined organic extracts were extracted repeatedly with dilute aqueous sodium hydroxide (pH 8). The combined aqueous basic layers were acidified with 6N hydrochloric acid. With cooling and scratching, a solid crystallised from solution. Suction filtration gave the product as a light brown powder (8.2 g, 60% yield based on the pyrone.

NMR ($CDCl_3$): 7.5 ppm (multiplet, 4H); 4.0 (S, 3H); 2.3 (S, 3H).

IR ($CH_2Cl_2$): 5.75µ, 6.22, 6.90. mp 203—204° (dec.). An analytical sample was crystallized from methanol.

Elemental Analysis

| | | | |
|---|---|---|---|
| Calculated: | C: 52.10; | H: 3.44; | N, 8.68 |
| Found: | C: 52.06; | H: 3.43; | N: 8.89 |

Example 2

Synthesis of Dimethyl-1-(p-chlorophenyl)-1,4-dihydro-4-oxo-6-methyl pyridazine-3,5-dicarboxylate (Part B) from dimethyl-2,3-dioxoglutarate 2-p-chlorophenyl hydrazone (Part A)

*Part (A).* A 10-liter widemouthed polyethylene container was fitted with a stirrer paddle and addition funnel. This container was charged with dimethyl-3-oxogluterate (1 kg, 5.75 moles), methanol (1.5 l), and sodium acetate (1 kg, 12.19 moles). p-Chlorobenzenediazonium chloride (5.75 moles) was generated on the side in seven equal portions, by combining p-chloroaniline (7 × 104 g, 5.75 moles), hydrochloric acid (7 × 314 ml 12N, 26 moles), water (7 × 200 ml) and sodium nitrite (7 × 65.6 g in 100 ml $H_2O$, 6.66 moles). The diazonium salt was added dropwise, rapidly to the reaction kettle. The pH was monitored periodically and maintained at 5 during the course of the reaction by adding sodium acetate *via* a spatula. At the end of the procedure, another 800 g of sodium acetate had been added. The resulting mixture was allowed to stand overnight, then suction filtered and the filter cake washed thoroughly with water. The brick-red filter cake was air dried to give 1.4 kg of the desired product (80% yield). NMR ($CDCl_3$): 7.5 ppm (S, 6H); 3.9 ppm (S, 6H); 3.7 ppm (S, 2H). This compound is known to the literature. See: Bulow and Hopfner. Berichte, *34,* 71 (901); ibid, *44,* 2835 (1911).

*Part (B).* A dry 1-liter, four-necked round bottomed flask was fitted with stirrer, thermometer, nitrogen inlet and rubber septum. The flask was charged with dimethyl-2,3-dioxoglutarate 2-p-chlorphenyl hydrazone (50 g, 0.16 mole) in dry tetrahydrofuran (170 ml). This solution was maintained under an atmosphere of dry nitrogen while cooling to 5°C. Isopropyl magnesium chloride (72 ml, 2.25 N in ethyl ether, 0.16 mole) was added dropwise *via* syringe, maintaining the pot temperature at 5—10°C. After complete addition, the mixture was stirred for 15 min. in the cold, then acetyl chloride (12 ml, 13.0 g, 0.16 mole) was added dropwise, rapidly, keeping the temperature of the reaction mixture below 10°C. The resulting dark solution was allowed to warm to room temperature during 2 h. Water (200 ml) was added. This mixture was stirred for 30 min., then extracted with ethyl acetate. The extracts were dried over $MgSO_4$, then filtered and reduced in volume *in vacuo.* The resulting dark oil was dissolved in ethyl ether, and cooled in an ice bath. The desired diester crystallized out to give 22.1 g yellow powder (42% yield), mp 153—54°C. NMR ($CDCl_3$): 7.6 ppm (multiplet, 4H); 4.0 ppm (S, 6H); 2.3 ppm (S, 3H). IR ($CH_2Cl_2$): 5.75μ, 6.12, 9.15.

Table I and II below give the structure, melting point in degrees Celsius and the elemental analysis of representative compounds of Formula (I) which were prepared by the procedures disclosed above. In Table II, the figures first appearing for elemental analysis are those calculated and those immediately thereafter are those found.

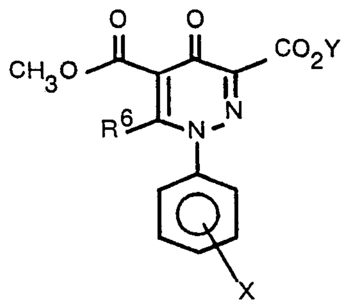

TABLE I

| Compound No. | X | Y | $R^6$ |
|---|---|---|---|
| 1 | 4-I | Na | $CH_3CH_2$ |
| 2 | 2-Cl | $CH_3$ | $CH_3CH_2$ |
| 3 | 4-I | $CH_3$ | $CH_3CH_2$ |
| 5 | 2,5-di$CH_3$ | $CH_3$ | $CH_3$ |
| 6 | 2,5-di$CH_3$ | Na | $CH_3CH_2$ |
| 7 | 2,5-di$CH_3$ | Na | $CH_3$ |
| 8 | 4-Cl | Na | $C_6H_5$ |
| 9 | 4-Cl | $CH_3$ | $C_6H_5$ |
| 10 | 4-Cl | Na | $CH_3CH_2CH_2CH_2$ |
| 11 | 4-Cl | $CH_3$ | $CH_3CH_2CH_2CH_2$ |
| 12 | 4-Cl | Na | $CH_3CH_2CH_2CH_2$ |
| 13 | 3,4-diCl | $CH_3$ | $CH_3CH_2$ |
| 14 | 4-Cl | $CH_3$ | *n*-propyl |
| 15 | H | Na | Isopropyl |
| 16 | H | $CH_3$ | Isopropyl |
| 17 | H | Na | $CH_3CH_2$ |
| 18 | H | $CH_3$ | $CH_3CH_2$ |
| 19 | 4-$CF_3$ | Na | $CH_3$ |
| 20 | 4-F | Na | $CH_3$ |
| 21 | 4-$CH_3$ | Na | $CH_3$ |
| 22 | 4-Cl | NH—$CH_2CH_2CH_2CH_3$ | $CH_3CH_2$ |
| 23 | 4-Br | Na | $CH_3$ |
| 24 | 4-I | Na | $CH_3$ |
| 25 | 3,4-diCl | Na | $CH_3$ |

TABLE I (Continued)

| Compound No. | X | Y | $R^6$ |
|---|---|---|---|
| 26 | H | Na | $CH_3$ |
| 27 | 4-Cl | Na | $CH_3$ |
| 28 | 4-Br | $CH_3CH_2CH_2$ | $CH_3$ |
| 29 | 4-Br | $CH_3$ | $CH_3$ |
| 30 | 4-Br | $CH_3CH_2$ | $CH_3$ |
| 31 | H | $CH_3$ | $CH_3$ |
| 32 | 4-Br | Na | $CH_3CH_2$ |
| 33 | 4-Br | $CH_3$ | $CH_3CH_2$ |
| 34 | 4-Cl | $CH_3$ | $CH_3CH_2$ |
| 35 | 4-Cl | Na | $CH_3CH_2$ |
| 36 | 2-Cl | Na | $CH_3CH_2$ |
| 37 | 4-Cl | $CH_3$ | $CH_3$ |

TABLE II

| Compound No. | mp(°C)[a] | Elemental Analysis[a] C | H | N |
|---|---|---|---|---|
| 1 | 225—30°(dec) | 42.07<br>42.48 | 3.06<br>3.01 | 6.54<br>7.11 |
| 2 | 130—31° | 54.78<br>54.57 | 4.31<br>4.15 | 7.99<br>8.39 |
| 3 | 210—11° | 43.45<br>43.57 | 3.42<br>3.39 | 6.34<br>6.66 |
| 5 | 157—58° | 61.81<br>61.73 | 5.49<br>5.52 | 8.48<br>8.85 |
| 6 | 221—22° | 61.81<br>62.04 | 5.49<br>5.69 | 8.48<br>8.74 |
| 7 | 205—207° | 60.75<br>60.72 | 5.10<br>5.23 | 8.86<br>9.26 |
| 8 | 193—94° | 59.31<br>59.37 | 3.41<br>3.45 | 7.28<br>7.03 |
| 9 | 216—17° | 60.23<br>60.19 | 3.79<br>3.77 | 7.03<br>6.75 |
| 10 | 153—54° | 55.97<br>55.74 | 4.70<br>4.66 | 7.68<br>7.33 |
| 11 | 109—11° | 57.07<br>56.86 | 5.06<br>5.03 | 7.40<br>7.21 |

TABLE II (Continued)

| Compound No. | mp(°C)[a] | Elemental Analysis[a] C | H | N |
|---|---|---|---|---|
| 12 | 205—6° | 54.78<br>54.79 | 4.31<br>4.24 | 7.99<br>7.96 |
| 13 | 154—55° | 50.15<br>50.03 | 3.68<br>3.55 | 7.31<br>7.58 |
| 14 | 141—42° | 55.97<br>56.01 | 4.69<br>4.75 | 7.68<br>7.54 |
| 15 | 209—11° (dec) | 60.75<br>61.30 | 5.09<br>5.21 | 8.86<br>9.40 |
| 16 | 158—59° | 61.81<br>61.61 | 5.49<br>5.51 | 8.48<br>8.30 |
| 17 | 194—95° | 59.59<br>58.93 | 4.67<br>4.58 | 9.27<br>9.78 |
| 18 | 187—88° | 60.75<br>60.46 | 5.09<br>5.14 | 8.86<br>8.78 |
| 19 | 206—08° | 50.57<br>50.40 | 3.11<br>3.03 | 7.87<br>8.18 |
| 20 | 209—10° | 54.90<br>54.90 | 3.62<br>3.67 | 9.15<br>9.13 |
| 21 | 216—18° | 59.60<br>59.43 | 4.67<br>4.78 | 9.27<br>9.17 |
| 22 | 127—28° | 57.22<br>57.28 | 5.33<br>5.31 | 11.12<br>10.66 |
| 23 | 215—17° | 45.79<br>45.71 | 3.02<br>3.02 | 7.63<br>7.58 |
| 24 | 228—31° | 40.59<br>40.60 | 2.68<br>2.68 | 6.77<br>6.70 |
| 25 | 218—19° | 47.08<br>47.22 | 2.82<br>2.93 | 7.85<br>7.98 |
| 26 | 223—27° | 58.33<br>58.05 | 4.20<br>4.21 | 9.72<br>9.59 |
| 27 | 203—4° | 52.10<br>52.06 | 3.44<br>3.43 | 8.68<br>8.89 |
| 28 | 122—24° | 49.89<br>50.10 | 4.19<br>4.33 | 6.85<br>6.85 |
| 29 | 163—65° | 47.26<br>47.26 | 3.44<br>3.40 | 7.35<br>7.49 |
| 30 | 151—52° | 48.62<br>48.82 | 3.83<br>3.77 | 7.09<br>7.47 |
| 31 | 183—84° | 59.60<br>59.87 | 4.67<br>4.72 | 9.27<br>9.24 |

TABLE II (Continued)

| Compound No. | mp(°C)[a] | Elemental Analysis[a] C | H | N |
|---|---|---|---|---|
| 32 | 199—200° | 47.26 | 3.44 | 7.35 |
| | | 47.52 | 3.41 | 7.56 |
| 33 | 155—57° | 48.62 | 3.83 | 7.09 |
| | | 48.79 | 3.87 | 6.94 |
| 34 | 138—39° | 54.78 | 4.31 | 7.99 |
| | | 55.36 | 4.36 | 8.20 |
| 35 | 219—20° | 53.49 | 3.89 | 8.32 |
| | | 53.65 | 3.88 | 8.54 |

(a) Melting points and elemental analyses recorded for the sodium salts are for the parent acid where Y is hydrogen.

The compounds of Formula (I) are particularly useful as chemical hybridization agents in gramineous crops, such as wheat, barley, maize, rice, sorghum, millets, oats, rye, triticale, forage crops and the like. When used as chemical hybridization agents, the compounds induce a selective male sterility without also inducing unacceptable female sterility in the treated plants and without causing unacceptable phytotoxicity towards the treated plants. As used herein, the term male sterility includes both actual male sterility, as evidenced by a lack of male flower parts or by sterile pollen, and functional male sterility, in which the male flower parts are unable to cause pollination. The compounds also cause other plant growth regulatory responses, such as, for example, control of flowering, control of fruiting and inhibition of seed formation in non-cereal species and other related growth regulatory responses.

Where, in the compounds of Formula (I), male sterility is accompanied by some female infertility and/or phytotoxicity, the compounds are still of utility in producing new plant hybrids or in the production of ergot for which see French Published Patent Application No. 2400832.

The compounds of Formula (I) may be used as plant growth regulators in the manner and at doses described in Belgian Patent No. 864,704 and its foreign equivalents listed earlier. The compounds of Formula I may also be formulated, with such changes as are dictated by the solubities and other characteristics of the compounds in question, as described in the Belgian Patent or its equivalents. The compounds of Formula (I) may be used singly or in admixture or may be used in conjunction with other types of plant growth regulators or with different types of agricultural chemicals as described in the Belgian patent and its equivalents.

Formulations usually include one or more of the compounds of Formula (I), e.g. in an amount from 1—99% by weight, and an agronomically acceptable carrier or diluent.

Of course, reference in the last three paragraphs — and elsewhere in this specification unless the context requires otherwise — to compounds of Formula (I) includes the acid addition salts.

In the biological data given later units are expressed in U.S. units, the metric equivalents of which are given below together with other explanatory information.

6 inches = 15.24 cm.
Parts are parts by weight.
Fertilizer (16—25—16) = (16—25—16 by wt. NPK).
1 tsp/gal = 1.3 ml/l.
* Isotox = benzenehexachloride.
Feekes' scale is described by W. Feekes, Vers. 17 Tech. Tarwe Comm, Groningen, pp 560, 561, 1941.
50 gal/A (acre) = 467.5 l/ha.
2 oz/50 gal = 56.7 g/189.25 l.
Dose = dose of active ingredient.

* (Isotox is a registered trademark).

| lbs/acre = | kg/ha | lbs/acre = | kg/ha |
|---|---|---|---|
| 1/128 | 0.009 | 1/2 | 0.561 |
| 1/64 | 0.017 | 1 | 1.121 |
| 1/32 | 0.035 | 2 | 2.242 |
| 1/16 | 0.07 | 4 | 4.484 |
| 1/8 | 0.140 | 8 | 8.968 |
| 1/4 | 0.280 | | |

Chemical Hybridization Activity

The following procedures are used to evaluate the activity of the compounds of the invention for inducing male sterility in cereals.

An awned variety (Fielder) and an awnless variety (Mayo-64) of spring wheat are planted at the rate of 6 to 8 seeds per inch pot containing a sterile medium of 3 parts soil and 1 part humus. The plants are grown under short-day (9 hour) conditions for the first 4 weeks to obtain good vegetative growth before flower initiation. The plants are then moved to long-day (16 hour) conditions which are provided by high intensity lights in the greenhouse. The plants are fertilized at 2, 4, and 8 weeks after planting with a water soluble fertilizer (16—25—16) at the rate of 1 tsp/gal of water, and are frequently sprayed with isotox for aphid control and dusted with sulfur for powdery mildew control.

Test compounds are foliarly applied to the awned female plants when these plants reach the flag leaf emergence stage (stage 8 on Feekes' scale). All compounds are applied in various concentrations in an aqueous carrier (containing if necessary, an organic solvent and conventional surfactant). The carrier volume is 50 gal/A (acre) in all cases. A typical usable surfactant is that sold as TRITON (registered trade mark) X100 used at a rate of 2 oz/50 gal of carrier.

After spike emergence but before anthesis, 4 to 6 spikes per pot are bagged to prevent outcrossing. At the first signs of flower opening, two spikes per pot are cross pollinated, using the approach method, with the awnless male parent. As soon as the seeds become plainly visible, spike length is measured and seeds per spikelet counted in both bagged and crossed spikes. Male sterility can then be calculated as percent inhibition of seed set in bagged spikes of treated plants, and female fertility in crossed spikes can be calculated as percent of control seed set. After maturity the seed on crossed spikes can be planted for determination of percent hybridization.

Where applicable, percent sterility, percent fertility, and percent height inhibition are calculated from the following formulas:

a. % Sterility = $(S_c - S_t/S_c) \times 100$
$S_c$ = seeds/spikelet in bagged spikes of control plants.
$S_t$ = seeds/spikelet in bagged spikes of treated plants.

b. % Fertility = $(F_t/F_c) \times 100$
$F_t$ = seeds/spikelet in approach crossed spikes of treated plants.
$F_c$ = seeds/spikelet in unbagged spikes of control plants.

c. % Height inhibition = $(H_c - H_t/H_c) \times 100$
$H_c$ = Height of control plants.
$H_t$ = Height of treated plants.

Table III summarizes typical results obtained in the evaluation of compounds of Formula I. A dash indicates that no determination of value was made.

TABLE III

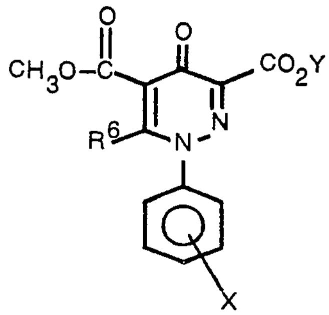

| Compound No. | X | Y | $R^6$ | Dose lb/A | Percent Male Sterility | Percent Female Sterility | Plant Injury 0—9 |
|---|---|---|---|---|---|---|---|
| 1 | 4-I | Na | $CH_3CH_2$ | 1/32 | 100 | — | trace |
| | | | | 1/8 | 100 | — | trace |
| | | | | 1/2 | 100 | — | 2 |
| | | | | 2 | 100 | — | 5 |
| 2 | 2-Cl | $CH_3$ | $CH_3CH_2$ | 1/8 | 57.0 | — | 0 |
| | | | | 1/2 | 100 | — | 0 |
| | | | | 2 | 100 | — | 1 |
| | | | | 8 | 100 | — | 4 |
| 3 | 4-I | $CH_3$ | $CH_3CH_2$ | 1/32 | 84.3 | — | 0 |
| | | | | 1/8 | 100 | — | 1 |
| | | | | 1/2 | 100 | — | 3 |
| | | | | 2 | 100 | — | 5 |
| 11 | 4-Cl | $CH_3$ | n-butyl | 1/8 | 19.0 | — | 0 |
| | | | | 1/2 | 16.0 | — | 0 |
| | | | | 2 | 32.7 | 620 | 0 |
| | | | | 8 | 87.8 | 7.6 | trace |
| 12 | 4-Cl | Na | n—$CH_3$—$CH_2CH_2$ | 1/8 | 80.2 | — | 3 |
| | | | | 1/2 | 100 | — | 5 |
| | | | | 2 | 100 | — | 7 |
| | | | | 8 | 100 | — | 8 |
| 13 | 3,4-diCl | $CH_3$ | $CH_3CH_2$ | 1/128 | 0 | — | 0 |
| | | | | 1/64 | 9.8 | 23.9 | 0 |
| | | | | 1/32 | 42.7 | 56.3 | 0 |
| | | | | 1/16 | 87.5 | 63.5 | 0 |

TABLE III (continued)

| Compound No. | X | Y | $R^6$ | Dose lb/A | Percent Male Sterility | Percent Female Sterility | Plant Injury 0—9 |
|---|---|---|---|---|---|---|---|
| 15 | H | Na | Isopropyl | 1/8 | 4.4 | — | 0 |
| | | | | 1/2 | 99.6 | — | 0 |
| | | | | 2 | 100 | — | 0 |
| | | | | 8 | 100 | — | 4 |
| 16 | H | $CH_3$ | Isopropyl | 1/8 | 21.8 | — | 0 |
| | | | | 1/2 | 79.9 | 40.2 | 0 |
| | | | | 2 | 100 | 0.43 | 0 |
| | | | | 8 | 100 | 0 | 2 |
| 17 | H | Na | $CH_3CH_2$ | 1/8 | 71.1 | — | 0 |
| | | | | 1/2 | 100 | — | 2 |
| | | | | 2 | 100 | — | 6 |
| | | | | 8 | — | — | 8 |
| 18 | H | $CH_3$ | $CH_3CH_2$ | 1/8 | 84.6 | — | — |
| | | | | 1/2 | 100 | — | — |
| | | | | 2 | 100 | — | — |
| | | | | 8 | — | — | — |
| 19 | 4-$CF_3$ | Na | $CH_3$ | 1 | 3.0 | — | 0 |
| | | | | 2 | 5.0 | — | 0 |
| | | | | 4 | 53.0 | — | 0 |
| | | | | 8 | 62.0 | — | 0 |
| 20 | 4-F. | Na | $CH_3$ | 1/8 | 0 | — | 0 |
| | | | | 1/4 | 0 | — | 0 |
| | | | | 1/2 | 7.0 | — | 0 |
| | | | | 1 | 83.0 | — | 0 |
| 22 | 4-Cl | CONH—$C_4H_9n$* | $CH_3CH_2$ | 1/8 | 4.0 | — | 0 |
| | | | | 1/2 | 4.4 | — | 0 |
| | | | | 2 | 86.7 | 69.4 | 0 |
| | | | | 8 | 100 | — | trace |

*Replaces COOY in the above general formula

TABLE III (continued)

| Compound No. | X | Y | $R^6$ | Dose lb/A | Percent Male Sterility | Percent Female Sterility | Plant Injury 0—9 |
|---|---|---|---|---|---|---|---|
| 23 | 4-Br | Na | $CH_3$ | 1/2 | 22.0 | — | 0 |
| | | | | 1 | 87.0 | — | trace |
| | | | | 2 | 100 | — | 3 |
| | | | | 4 | 100 | — | 5 |
| 24 | 4-I | Na | $CH_3$ | 1/2 | 0 | — | 0 |
| | | | | 1 | 72.0 | — | 0 |
| | | | | 2 | 99.0 | — | 0 |
| | | | | 4 | 100 | — | 2 |
| 25 | 3,4 diCl | Na | $CH_3$ | 1/2 | 3.0 | — | 0 |
| | | | | 1 | 2.0 | — | 0 |
| | | | | 2 | 43.0 | — | trace |
| | | | | 4 | 70.0 | — | 3 |
| 26 | H | Na | $CH_3$ | 1/2 | 12.0 | — | 0 |
| | | | | 1 | 77.0 | — | 0 |
| | | | | 2 | 92.0 | — | 0 |
| | | | | 4 | 100 | — | 0 |
| 27 | 4-Cl | Na | $CH_3$ | 1/2 | 4.0 | — | 0 |
| | | | | 1 | 84.0 | — | 0 |
| | | | | 2 | 100 | — | 0 |
| | | | | 4 | 94.0 | — | 1 |
| 28 | 4-Br | $CH_3—CH_2CH_2$ | $CH_3$ | 1 | 10.0 | — | 0 |
| | | | | 2 | 57.0 | — | 0 |
| | | | | 4 | 93.0 | — | 1/2 |
| | | | | 8 | 100 | — | 2 |
| 29 | 4-Br | $CH_3$ | $CH_3$ | 1/8 | 0 | — | 0 |
| | | | | 1/4 | 27.0 | — | 0 |
| | | | | 1/2 | 80.0 | — | 0 |
| | | | | 1 | 100 | — | 1/2 |
| 30 | 4-Br | $CH_3CH_2$ | $CH_3$ | 1 | 9.0 | — | 0 |
| | | | | 2 | 64.0 | — | 0 |
| | | | | 4 | 99.0 | — | 2 |
| | | | | 8 | 100 | — | 3 |

TABLE III (continued)

| Compound No. | X | Y | $R^6$ | Dose lb/A | Percent Male Sterility | Percent Female Sterility | Plant Injury 0—9 |
|---|---|---|---|---|---|---|---|
| 31 | H | $CH_3$ | $CH_3$ | 1/8 | 0 | — | 0 |
| | | | | 1/2 | 80 | — | 0 |
| | | | | 2 | 100 | — | 0 |
| | | | | 8 | 100 | — | 1 |
| 32 | 4-Br | Na | $CH_3CH_2$ | 1/32 | 11.4 | — | 0 |
| | | | | 1/16 | 69.3 | — | 0 |
| | | | | 1/8 | 99.1 | — | 0 |
| | | | | 1/4 | 100 | — | 2 |
| 33 | 4-Br | $CH_3$ | $CH_3CH_2$ | 1/32 | 91.2 | — | 0 |
| | | | | 1/16 | 85.1 | — | 0 |
| | | | | 1/8 | 100 | — | 0 |
| | | | | 1/4 | 100 | — | 0 |
| 34 | 4-Cl | $CH_3$ | $CH_3CH_2$ | 1/32 | 40.9 | — | 0 |
| | | | | 1/16 | 92.2 | — | 1 |
| | | | | 1/8 | 94.6 | — | 2 |
| | | | | 1/4 | 95.5 | — | 3 |
| 35 | 4-Cl | Na | $CH_3CH_2$ | 1/32 | 36.4 | — | 1 |
| | | | | 1/16 | 100 | — | 2 |
| | | | | 1/8 | 91.3 | — | 1 |
| | | | | 1/4 | 100 | — | 2 |
| 36 | 2-Cl | Na | $C_2H_5$ | 1/8 | 100 | — | 0 |
| | | | | 1/2 | 100 | — | trace |
| | | | | 2 | 100 | — | 3 |
| | | | | 8 | 100 | — | 4 |
| 37 | 4-Cl | $CH_3$ | $CH_3$ | 1/8 | 4 | — | — |
| | | | | 1/4 | 0 | — | — |
| | | | | 1/2 | 53 | — | — |
| | | | | 1 | 69 | — | — |
| | | | | 2 | 92 | — | — |

**Claims for the Contracting States: BE CH DE FR GB IT LI NL SE**

1. A compound of Formula (I) below, or an agronomically acceptable acid addition salt thereof,

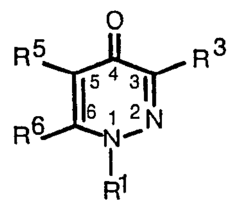

(I)

wherein $R^1$ is ($C_1$—$C_4$)alkyl, phenyl or naphthyl each of the last two groups being optionally substituted with up to three of the same or different substituents selected from halogen, trihalomethyl, ($C_1$—$C_4$)alkoxy, ($C_1$—$C_4$)alkyl and cyano;

$R^3$ is a group as defined below for $R^5$;

$R^5$ is a carboxy (—COOH) group or agronomically acceptable alkali metal salt thereof, an alkoxycarbonyl (—COOR) group or substituted carbamoyl (—CONR'R'') group, wherein R represents ($C_1$—$C_4$)alkyl, R' represents hydrogen or ($C_1$—$C_4$)alkyl and R'' represents ($C_1$—$C_4$) alkyl; and $R^6$ is a ($C_1$—$C_4$) alkyl group or an optionally substituted phenyl or naphthyl group as defined for $R^1$ above: provided that where $R^1$ is unsubstituted phenyl, $R^6$ is not 2-methoxyphenyl.

2. A compound as claimed in Claim 1, wherein $R^1$ is halo-substituted phenyl, $R^3$ is $CO_2Na$, $CO_2K$ or $CO_2CH_3$, $R^5$ is $CO_2CH_3$ and $R^6$ is methyl or ethyl.

3. A compound as claimed in Claim 1, wherein $R^1$ is 4-chloro- or 4-bromo-phenyl, $R^3$ is $CO_2Na$ or $CO_2K$, $R^5$ is $CO_2CH_3$ and $R^6$ is ethyl.

4. A plant growth regulating composition containing at least one compound as claimed in any preceding claim and an agronomically acceptable carrier therefor.

5. A method of regulating the growth of a gramineous crop plant which comprises applying to the growing plant, or to seeds thereof, a growth regulating amount of one or more compounds or compositions claimed in any one of Claims 1—4.

6. A method as claimed in Claim 5 as applied to the production of hydbrid seed by using at least one growth regulating compound which induces male sterility without destroying female fertility.

7. A method as claimed in Claim 6 wherein the plant is maize, barley, wheat, sorghum or a forage crop plant.

**Claims for the Contracting State: AT**

1. A plant growth regulating composition containing an active plant growth regulation component and an agronomically acceptable diluent or carrier therefor, wherein the active component comprises at least one compound of Formula (I) below, or an agronomically acceptable acid addition salt thereof,

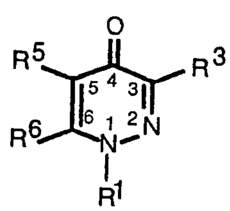

(I)

wherein $R^1$ is ($C_1$—$C_4$)alkyl, phenyl or naphthyl each of the last two groups being optionally substituted with up to three of the same or different substituents selected from halogen, trihalomethyl, ($C_1$—$C_4$)alkoxy, ($C_1$—$C_4$)alkyl and cyano;

$R^3$ is a group as defined below for $R^5$;

$R^5$ is a carboxy (—COOH) group or agronomically acceptable alkali metal salt thereof, an alkoxycarbonyl (—COOR) group or substituted carbamoyl (—CONR'R'') group, wherein R represents ($C_1$—$C_4$) alkyl, R' represents hydrogen or ($C_1$—$C_4$) alkyl, and R'' represents ($C_1$—$C_4$) alkyl; and $R^6$ is a ($C_1$—$C_4$) alkyl group or an optionally substituted phenyl or naphthyl group as defined for $R^1$ above: provided that where $R_1$ is unsubstituted phenyl, $R^6$ is not 2-methoxyphenyl.

2. A composition as claimed in Claim 1, wherein $R^1$ is halo-substituted phenyl, $R^3$ is $CO_2Na$, $CO_2K$ or $CO_2CH_3$, $R^5$ is $CO_2CH_3$ and $R^6$ is methyl or ethyl.

3. A composition as claimed in Claim 2, wherein $R^1$ is 4-chloro- or 4-bromo-phenyl, $R^3$ is $CO_2Na$ or $CO_2K$, $R^5$ is $CO_2CH_3$ and $R^6$ is ethyl.

4. A method of regulating the growth of a gramineous crop plant which comprises applying to the

growing plant, or to seeds thereof, a growth regulating amount of a composition claimed in any one of Claims 1—3.

5. A method as claimed in Claim 4 as applied to the production of hybrid seed by using at least one growth regulating composition which induces male sterility without destroying female fertility.

6. A method as claimed in Claim 5 wherein the plant is maize, barley, wheat, sorghum or a forage crop plant.

**Patentansprüche für die Vertragsstaaten: BE CH DE FR GB IT LI NL SE**

1. Verbindung der Formel I

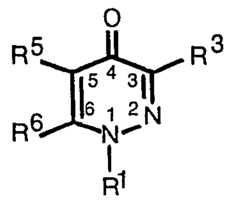

(I)

oder ein agronomisch zulässiges Säureadditionssalz davon, worin

$R^1$ für $(C_1—C_4)$Alkyl, Phenyl oder Naphthyl steht, wobei jede der letzten beiden Gruppen gegebenenfalls mit bis zu drei gleichen oder verschiedenen Substituenten substituiert ist, die ausgewählt sind aus Halogen, Trihalogenmethyl, $(C_1—C_4)$Alkoxy, $(C_1—C_4)$Alkyl und Cyano;

$R^3$ für eine Gruppe steht, wie sie unten für $R^5$ definiert ist;

$R^5$ für eine Carboxy-Gruppe (—COOH) oder ein agronomisch zulässiges Alkalimetallsalz davon, eine Alkoxycarbonyl-Gruppe (—COOR) oder eine substituierte Carbamoyl-Gruppe (—CONR'R'') steht, worin R für $(C_1—C_4)$Alkyl, R' für Wasserstoff oder $(C_1—C_4)$Alkyl und R'' für $(C_1—C_4)$Alkyl steht; und

$R^6$ für eine $(C_1—C_4)$Alkyl-Gruppe oder eine gegebenenfals substituierte Phenyl- oder Naphthyl-Gruppe, wie sie oben für $R^1$ definiert ist, steht;

mit der Maßgabe, daß, wenn $R^1$ unsubstituiertes Phenyl bedeutet, $R^6$ nicht für 2-Methoxyphenyl steht.

2. Verbindung nach Anspruch 1, worin $R^1$ für halogen-substituiertes Phenyl, $R^3$ für $CO_2Na$, $CO_2K$ oder $CO_2CH_3$, $R^5$ für $CO_2CH_3$ und $R^6$ für Methyl oder Ethyl steht.

3. Verbindung nach Anspruch 1, worin $R^1$ für 4-Chloro- oder 4-Bromo-phenyl, $R^3$ für $CO_2Na$ oder $CO_2K$, $R^5$ für $CO_2CH_3$ und $R^6$ für Ethyl steht.

4. Das Pflanzenwachstum regulierende Zusammensetzung, welche mindestens eine Verbindung nach einem der vorhergehenden Ansprüche und einen agronomisch zulässigen Träger hierfür enthält.

5. Verfahren zur Regulierung des Wachstums einer grasartigen Kulturpflanze, bei welchem auf die wachsende Pflanze oder auf deren Samen eine das Wachstum regulierende Menge einer oder mehrer Verbindungen oder Zusammensetzungen nach einem der Ansprüche 1 bis 4 aufgebracht wird.

6. Verfahren nach Anspruch 5 in der Anwendung auf die Erzeugung von Hybridsamen, wobei mindestens eine das Wachstum regulierende Verbindung verwendet wird, welche eine männliche Sterilität erzeugt, ohne daß sie die weibliche Fertilität zerstört.

7. Verfahren nach Anspruch 6, bei welchem als Pflanze Mais, Gerste, Weizen, Sorghum oder eine Futterkulturpflanze verwendet wird.

**Patentansprüche für den Vertragsstaat: AT**

1. Das Planzenwachstum regulierende Zusammensetzung, welche eine aktive, das Pflanzenwachstum regulierende Komponente und ein agronomisch zulässiges Verdünnungs- oder Trägermittel hierfür enthält, bei welcher die aktive Komponente aus mindestens einer Verbindung der Formel I

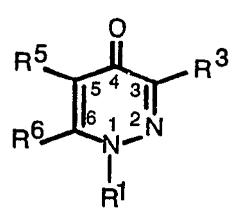

(I)

oder einem agronomisch zulässigen Säureadditionssalz davon besteht, worin

$R^1$ für $(C_1—C_4)$Alkyl, Phenyl oder Naphthyl steht, webei jede der letzten beiden Gruppen gegebenenfalls mit bis zu drei gleichen oder verschiedenen Substituenten substituiert ist, die ausgewählt sind aus Halogen, Trihalogenmethyl, $(C_1—C_4)$Alkoxy, $(C_1—C_4)$Alkyl und Cyano;

$R^3$ für eine Gruppe steht, wie sie unten für $R^5$ definiert ist;

$R^5$ für eine Carboxy-Gruppe (—COOH) oder ein agronomisch zulässiges Alkalimetallsalz davon, eine Alkoxycarbonyl-Gruppe (—COOR) oder eine substituierte Carbamoyl-Gruppe (—CONR'R'') steht, worin R für ($C_1$—$C_4$)Alkyl, R' für Wasserstoff oder ($C_1$—$C_4$)Alkyl und R'' für ($C_1$—$C_4$)Alkyl steht; und

$R^6$ für eine ($C_1$—$C_4$)Alkyl-Gruppe oder eine gegebenenfals substituierte Phenyl- oder Naphthyl-Gruppe, wie sie oben für $R^1$ definiert ist, steht;

mit der Maßgabe, daß, wenn $R^1$ unsubstituiertes Phenyl bedeutet, $R^6$ nicht für 2-Methoxyphenyl steht.

2. Zusammensetzung nach Anspruch 1, worin $R^1$ für halogen-substituiertes Phenyl, $R^3$ für $CO_2Na$, $CO_2K$ oder $CO_2CH_3$, $R^5$ für $CO_2CH_3$ und $R^6$ für Methyl oder Ethyl steht.

3. Zusammensetzung nach Anspruch 2, worin $R^1$ für 4-Chloro- oder 4-Bromo-phenyl, $R^3$ für $CO_2Na$ oder $CO_2K$, $R^5$ für $CO_2CH_3$ und $R^6$ für Ethyl steht.

4. Verfahren zur Regulierung des Wachstums einer grasartigen Kulturpflanze, bei welchem auf die wachsende Pflanze oder auf den Samen eine das Wachstum reglierende Menge einer Zusammensetzung nach einem der Ansprüche 1 bis 3 aufgebracht wird.

5. Verfahren nach Anspruch 4 in der Anwendung auf die Erzeugung von Hybridsamen, wobei mindestens eine das Wachstum regulierende Verbindung verwendet wird, welche eine männliche Sterilität erzeugt, ohne daß sie die weibliche Fertilität zerstört.

6. Verfahren nach Anspruch 5, bei welchem als Pflanze Mais, Gerste, Weizen, Sorghum oder eine Futterkulturpflanze verwendet wird.

**Revendications pour les Etats contractants: BE CH DE FR GB IT LI NL SE**

1. Composé répondant à la formule (I) ci-dessous, ou un de ses sels d'addition d'acides acceptables du point de vue agronomique,

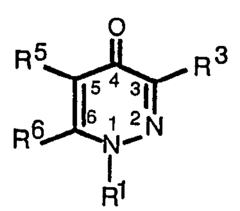

(I)

formule dans laquelle $R^1$ représente un groupe alkyle en $C_1$ à $C_4$, phényle ou naphtyle, chacun des deux derniers groupes étant facultativement substitué avec jusqu'à trois substituants identiques ou différents choisis entre des groupes halogéno, trihalogénométhyle, alkoxy en $C_1$ à $C_4$, alkyle en $C_1$ à $C_4$ et cyano;

$R_3$ représente un groupe répondant à la définition mentionnée ci-dessous pour $R^5$;

$R^5$ représente un groupe carboxy (—COOH) ou un de ses sels de métaux alcalins acceptables du point de vue agronomique, un groupe alkoxycarbonyle (—COOR) ou un groupe carbamoyle substitué (—CONR'R''), dans lequel R représente un groupe alkyle en $C_1$ à $C_4$, R' représente l'hydrogène ou un groupe alkyle en $C_1$ à $C_4$ et R'' représente un groupe alkyle en $C_1$ à $C_4$; et $R^6$ représente un groupe alkyle en $C_1$ à $C_4$ ou un groupe phényle ou naphtyle facultativement substitué, suivant la définition mentionnée ci-dessus pour $R^1$;

sous réserve que, lorsque $R^1$ représente un groupe phényle non substitué, $R^6$ ne représente pas un groupe 2-méthoxyphényle.

2. Composé suivant la revendication 1, dans lequel $R^1$ représente un groupe phényle substitué avec un halogène, $R^3$ représente un groupe $CO_2Na$, $CO_2K$ ou $CO_2CH_3$, $R^5$ représente un groupe $CO_2CH_3$ et $R^6$ représente un groupe méthyle ou éthyle.

3. Composé suivant la revendication 1, dans lequel $R^1$ représente un groupe 4-chloro- ou 4-bromophényle, $R^3$ représente un groupe $CO_2Na$ ou $CO_2K$, $R^5$ représente un groupe $CO_2CH_3$ et $R^6$ représente un groupe éthyle.

4. Composition régulatrice de croissance des plantes contenant au moins un composé suivant l'une quelconque des revendications précédentes et un support acceptable du point de vue agronomique.

5. Procédé de régulation de la croissance d'une graminée cultivée, qui consiste à appliquer à la plante en croissance, ou à ses graines, une quantité régulatrice de croissance d'un ou plusieurs composés ou d'une ou plusieurs compositions suivant l'une quelconque des revendications 1 à 4.

6. Procédé suivant la revendication 5, mis en oeuvre pour la production de semences hybrides, par l'utilisation d'au moins un composé régulateur de croissance qui induit une stérilité chez le sexe mâle sans supprimer la fertilité chez le sexe femelle.

7. Procédé suivant la revendication 6, dans lequel la plante est le maïs, l'orge, le blé, le sorgho ou une plante de culture fourragère.

**Revendications pour l'Etat contractant: AT**

1. Composition régulatrice de croissance des plantes contenant un constituant doué d'une activité de régulation de croissance des plantes et un diluant ou support acceptable du point de vue agronomique,

dans laquelle le constituant actif comprend au moins un composé répondant à la formule (I) ci-dessous, ou un de ses sels d'addition d'acides acceptables du point de vue agronomique,

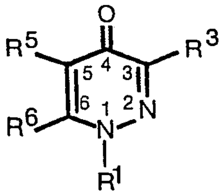

(I)

formule dans laquelle $R^1$ représente un groupe alkyle en $C_1$ à $C_4$, phényle ou naphtyle, chacun des deux derniers groupes étant facultativement substitué avec jusqu'à trois substituants identiques ou différents choisis entre des groupes halogéno, trihalogénométhyle, alkoxy en $C_1$ à $C_4$, alkyle en $C_1$ à $C_4$ et cyano;

$R_3$ représente un groupe répondant à la définition mentionnée ci-dessous pour $R^5$;

$R^5$ représente un groupe carboxy (—COOH) ou un de ses sels de métaux alcalins acceptables du point de vue agronomique, un groupe alkoxycarbonyle (—COOR) ou un groupe carbamoyle substitué (—CONR'R''), dans lequel R représente un groupe alkyle en $C_1$ à $C_4$, R' représente l'hydrogène ou un groupe alkyle en $C_1$ à $C_4$ et R'' représente un groupe alkyle en $C_1$ à $C_4$; et $R^6$ représente un groupe alkyle en $C_1$ à $C_4$ ou un groupe phényle ou naphtyle, facultativement substitué, suivant la définition mentionnée ci-dessus pour $R^1$;

sous réserve que, lorsque $R^1$ représente un groupe phényle non substitué, $R^6$ ne représente pas un groupe 2-méthoxyphényle.

2. Composition suivant la revendication 1, dans laquelle $R^1$ représente un groupe phényle substitué avec un halogéne, $R^3$ représente un groupe $CO_2Na$, $CO_2K$ ou $CO_2CH_3$, $R^5$ représente un groupe $CO_2CH_3$ et $R^6$ représente un groupe méthyle ou éthyle.

3. Composition suivant la revendication 2, dans laquelle $R^1$ représente un groupe 4-chloro- ou 4-bromophényle, $R^3$ représente un groupe $CO_2Na$ ou $CO_2K$, $R^5$ représente un groupe $CO_2CH_3$ et $R^6$ représente un groupe éthyle.

4. Procédé de régulation de la croissance d'une graminée cultivée, qui consiste à appliquer à la plante en croissance, ou à ses graines, une quantité régulatrice de croissance d'une composition suivant l'une quelconque des revendications 1 à 3.

5. Procédé suivant la revendication 4, mis en oeuvre pour la production de semences hybrides, par l'utilisation d'au moins une quantité régulatrice de croissance qui induit une stérilité chez le sexe mâle sans supprimer la fertilité chez le sexe femelle.

6. Procédé suivant la revendication 5, dans lequel la plante est le maïs, l'orge, le blé, le sorgho ou une plante de culture fourragère.